Europäisches Patentamt

European Patent Office

Office européen des brevets

(19)

(11) Publication number: **0 392 554**
**A1**

## EUROPEAN PATENT APPLICATION

(21) Application number: **90107085.4**

(22) Date of filing: **12.04.90**

(51) Int. Cl.⁵: **C07C 2/24, C07C 11/10, C07C 11/113**

(30) Priority: **14.04.89 US 338785**

(43) Date of publication of application:
**17.10.90 Bulletin 90/42**

(84) Designated Contracting States:
**AT BE CH DE ES FR GB GR IT LI LU NL SE**

(71) Applicant: **PHILLIPS PETROLEUM COMPANY**
**5th and Keeler**
**Bartlesville Oklahoma 74004(US)**

(72) Inventor: **Drake, Charles Alfred**
**Rt. 1 Box. 206**
**Nowata, OK 74048(US)**

(74) Representative: **Dost, Wolfgang,**
**Dr.rer.nat.,Dipl.-Chem. et al**
**Patent- & Rechtsanwälte Bardehle .**
**Pagenberg . Dost . Altenburg . Frohwitter .**
**Geissler & Partner Galileiplatz 1 Postfach 86**
**06 20**
**D-8000 München 86(DE)**

(54) **Olefin dimerization process.**

(57) A method to dimerize ethylene and propylene to form pentene(s) and hexene(s) is provided. The quantity of each of the reaction products can be controlled by varying the reactant ratio and/or reaction temperature. The catalyst used is an elemental alkali metal supported on an alkali metal carbonate; optional promoters can be used.

EP 0 392 554 A1

## OLEFIN DIMERIZATION PROCESS

## BACKGROUND OF THE INVENTION

This invention relates to olefin dimerization with alkali metal carbonate supported alkali metal catalysts.

It is known in the art to employ alkali metal carbonate supported elemental alkali metal catalysts for such conversions as olefin dimerization. Several processes for dimerizing olefins are also known in the art. In order to produce a chemical, such as an olefin, efficiently, it is necessary to construct as large of a commercial plant as possible. Unfortunately, each process and set of equipment, which includes, for example, catalyst, machinery, and/or reactants, is usually limited to yield only one desired product. Therefore, cost can be prohibitive. However, if one set of equipment, i.e., one commercial plant and one catalyst, can be used to produce more than one desired product, the cost effectiveness of such equipment and/or series of processes is greatly increased.

Propylene and ethylene can be dimerized in the presence of an elemental alkali metal supported alkali metal carbonate catalyst in accordance with the following equation:

$$3C_3H_6 + C_2H_4 \rightarrow C_5H_{10} + C_6H_{12}$$

Stoichiometrically, three moles of propylene react with one mole of ethylene to produce one mole of pentene(s) and one mole of hexene(s). The pentene(s) and hexene(s) can be one or more isomers of either molecule. Unfortunately, stoichiometric quantities of pentene(s) and hexene(s) are not always required or desired.

## SUMMARY OF THE INVENTION

It is an object of this invention to provide an improved process for the dimerization of olefins.

It is another object of this invention to provide an improved process to dimerize propylene and ethylene.

It is yet another object of this invention to provide an improved process to prepare pentene and/or 4-methyl-1-pentene.

In accordance with this invention, a process comprising contacting propylene and ethylene, under dimerization conditions, in the presence of a catalyst comprising at least one elemental alkali metal supported on an alkali metal carbonate to produce at least one mono-1-olefin reaction product, wherein the type and quantity of said reaction product can be controlled by varying the feed ratio of propylene to ethylene, and varying the dimerization conditions.

## DESCRIPTION OF THE PREFERRED EMBODIMENTS

### Supports

The catalyst support can be formed by any method known in the art. Thus, commercially available alkali metal carbonate in the form of powder, pellets, granules, or any other form can be treated directly with at least one elemental alkali metal and, optionally, one or more of the desired promoting materials as discussed more fully below. This form of support has the advantage of being most readily obtained with a minimum of handling.

In some circumstances, a large particle size and/or more rugged form of catalyst support is desired, such as, for example, where fixed bed reactions, especially large volume fixed bed reactions, are carried out. One particular technique for support preparation is to form a thick paste comprising alkali metal carbonate, water, and alcohol; or alkali metal carbonate, water, and water soluble ketone. The thick paste can be extruded, pelletized, pilled, or tabletted into appropriate sizes. The resultant material is then oven dried under conditions of time and temperature to insure that substantially all liquid is driven off. These types of supports will be referred to as "wet process" alkali metal carbonate supports.

Alcohols suitable for use in preparation of "wet process" catalyst supports are straight chain and branched aliphatic alcohols having from about 1 to about 7 carbon atoms. Water soluble ketones suitable for use in preparation of "wet process" catalyst supports are straight chain and branched water soluble ketones having from about 3 to about 7 carbon atoms.

In accordance with another technique for the support preparation alkali metal carbonate can be mixed with a non-acidic inorganic oxide and/or finely divided stainless steel. The mixture is heated to at least 950° C, then cooled, and finally, if desired, broken into pieces or fractionated to a desired particle size. Catalyst support prepared in this manner will be referred to as "melt process" alkali metal carbonate supports.

Suitable non-acidic inorganic oxides include, but are not limited to, alumina, such as alpha-alumina, silica, silica-alumina, magnesia-titania, thoria, magnesia, titania, zirconia, and mixtures of two or more thereof. Stainless steel as used herein is intended to cover broadly those alloys of iron which are relatively inert to the reaction conditions employed for olefin dimerization.

In accordance with another technique for the support preparation, an alkali metal carbonate is pelletized with at least one carbonaceous compound. The pelleted support, either as pellets or as smaller crushed particles, is then heated in an oxygen-containing atmosphere under conditions suitable to oxidize in the range of about 10 to about 90 weight percent of the carbonaceous compound. As a result of this partial oxidation of the pelleted support, the concentration of carbonaceous compound remaining on the surface of the support is substantially less than the concentration of carbonaceous compound remaining on the interior portions of the support. Catalyst support prepared in this manner will be referred to as "carbon containing" alkali metal carbonate support.

The term "carbonaceous compound" is intended to include various forms of the element carbon. Examples include, but are not limited to, carbon black, charcoal, coconut charcoal, amorphous graphite, and crystallite graphite.

Once the catalyst support is formed, it should be calcined in an oxygen-containing atmosphere at a temperature in the range of about 80° to about 350° C, preferably about 250° C, for a time of at least 2 hours. Times in excess of about 20 hours generally impart no additional beneficial effect. Thus, times in the range of about 2 to about 20 hours are useful. Upon completion of calcination, the catalyst support can be stored in a dry atmosphere. Preferably, the catalyst support is stored under a dry, oxygen-free atmosphere until needed for further treatment.

## Catalysts and Promoters

Catalysts systems employed in the practice of this invention comprise one of the alkali metal carbonate supports described above, at least one elemental alkali metal catalyst, and optionally one or more of the following additional promoters:
elemental copper,
elemental cobalt,
finely divided stainless steel,
finely divided glass, and
mixtures of two or more thereof.

It should be recognized, however, that the catalyst systems of the invention can contain additional components which do not adversely affect the catalyst performance, such as, for example, pigments, dyes, processing aids, inert fillers, binders and the like.

The alkali metals contemplated to be within the scope of the invention include lithium, sodium, potassium, rubidium and cesium. While the proportion of alkali metal combined with the alkali metal carbonate support can vary appreciably, generally at least about one weight percent of alkali metal based on the total weight of treated support will be employed. Generally, about 1 to about 20 weight percent alkali metal will be employed with about 2 to about 15 weight percent preferred. A n alkali metal loading of about 3 to about 10 weight percent based on the total weight of treated support is most preferred for most efficient use of reagents, high catalyst activity and selectivity, and ease of catalyst preparation. Potassium is the preferred elemental alkali metal due to its ready availability as well as ease and safety in handling.

The proportion of optional promoter on the alkali metal carbonate support can vary appreciably, but generally, at least one weight percent of the optional promoter based on the total weight of treated support will be employed. The following amounts are provided for additional guidance:

3

| Promoter | Broad | Intermediate | Preferred |
|----------|-------|--------------|-----------|
| Cu       | 1-30  | 3-20         | 5-12      |
| Co       | 1-50  | 3-25         | 5-15      |
| *SS      | 1-80  | 3-60         | 5-50      |
| Glass    | 1-50  | 2-25         | 3-15      |

*SS = Stainless Steel

The general procedure for preparation of the catalyst systems, after calcining the support, of the invention involves heating the alkali metal carbonate support to a temperature in the range of about 80° to about 350° C, preferably slightly above the melting point of the particular alkali metal used, cooling the particulate support and then contacting the particulate support with at least one elemental alkali metal in a dry, oxygen-free atmosphere, such as, for example $N_2$, Ar, or the like, at a temperature sufficient to cause the alkali metal to melt. The contacting, done in an oxygen-free atmosphere, is preferably carried out with suitable mixing to ensure even distribution. Suitable temperatures for the contacting step will vary with the particular alkali metal employed. For example, with elemental potassium, temperatures in the range of about 80° to 100° C are preferred, while with elemental sodium, temperatures in the range of about 100° to 140° C are preferred.

While the alkali metal treated support is maintained at or above the melting point of the particular alkali metal used, in an oxygen-free atmosphere, any desired promoter(s), such as for example, finely divided stainless steel or elemental copper, can be gradually added while the treated catalyst is continuously stirred. For example, with potassium, temperatures in the range of about 80° to about 100° C are employed. The catalyst system is then ready to be charged to the reactor.

Optionally, the alkali metal carbonate support, once elemental alkali metal and any desired promoters have been deposited thereon, can be subjected to a subsequent heating step, in an oxygen-free atmosphere, to ensure as uniform a distribution as possible of the various promoters on the surface of the alkali metal carbonate support. Thus, the finished catalyst can be subjected to a temperature in the range of at least about 80° C for a time in the range of about 0.1 to about 4 hours. A temperature in the range of about 150° to about 250° C for a time in the range of about 0.5 to about 2 hours is presently preferred for the most uniform distribution.

Optionally, prior to charging the reactor, the catalyst system can be mixed with an inert substance to dilute the catalyst system and decrease the rate of olefin dimerization. Any inert substance which has no catalytic activity in an olefin dimerization reaction can be used. One example of such an inert substance is glass beads.

As indicated by the variety of supports, alkali metal components, and promoters included within the scope of the invention, numerous catalyst combinations are possible. Any combination of the alkali metal and optional promoters disclosed can be supported on any alkali metal carbonate support disclosed. Some possible combinations are described in detail in the examples which follow. The combination of support, alkali metal and promoter(s) which one may choose to employ will depend on one or more variables, such as for example, reactor configuration, reaction temperature and pressure, olefin feed employed, rate of olefin feed, and conversions desired.

The resultant catalyst can be treated in any way known in the art, as long as the catalyst is not adversely affected. For example, the catalyst can be treated with a paraffinic material.


Reactants


Reactants applicable for use in the process of the invention are olefinic compounds which can (a) self-react, i.e., dimerize, to give useful products such as, for example, the self-reaction of propylene can give 4-methyl-1-pentene; and/or (b) olefinic compounds which can react with other olefinic compounds, i.e., co-dimerize, to give useful products such as, for example, co-dimerization of ethylene plus propylene can give 1-pentene. As used herein, the term "dimerization" is intended to include both self-reaction and "co-dimerization" as defined above.

The suitable dimerizable olefinic compound useful in this invention is propylene. Propylene, when dimerized, forms hexene(s) and isomers thereof. Exemplary hexene isomers include, but are not limited to,

4

1-hexene, 2-hexene, 3-hexene, 4-methyl-1-pentene, 4-methyl-2-pentene, and/or 2-methyl-2-pentene.

The suitable co-dimerizable olefinic compounds useful in this invention are ethylene and propylene. When ethylene and propylene are dimerized, pentene(s) and isomers thereof can be produced. Exemplary pentene isomers include, but are not limited to 1-pentene, 2-pentene, 2-methyl-1-butene, and/or 3-methyl-1-butene.

The quantity of each reactant used can vary in accordance with what product hexene(s) and/or pentene-(s), are desired. One method to express the quantity of each reactant used is to use a weight ratio. The weight ratio of propylene to ethylene usually is within the range of about 0.5:1 to about 15:1. If greater hexene(s) production is required, the propylene to ethylene ratio should be higher, preferably within the range of about 6:1 to about 15:1. If more pentene(s) are desired, a lower propylene to ethylene ratio should be used, preferably within the range of about 0.5:1 to about 6:1.

Another method to express the quantity of each reactant used is the mole ratio of propylene to ethylene. The mole ratio of propylene to ethylene is usually within the range of about 0.3:1 to about 10:1. If greater hexene(s) production is required, preferably the propylene to ethylene mole ratio is within the range of about 4:1 to about 10:1. If more pentene(s) are desired, the propylene to ethylene mole ratio preferably is within the range of about 0.3:1 to about 4:1.

## Reaction Conditions

The dimerization reaction of the invention can be carried out using either batch or continuous types of operation, although the catalysts of the invention are particularly well suited for continuous, fixed bed, operation. Suitable equipment such as for example autoclaves, tubular reactors and the like as are well known in the art can be employed. No special materials of construction are required so that steel, stainless steel, glass-lined reactors, or the like can be employed.

The reaction temperature can vary depending on the catalyst and feed rates and quantities of reactants employed. Typically, a temperature range of about 50° to about 250°C is suitable. Temperatures in the range of about 80° to about 200°C are preferred, with a range of about 130° to about 170°C most preferred because optimum reaction rates are obtained with minimum by-product formation. Surprisingly, it has been found that the temperature of the reactor affects the resultant products. Lower temperatures, preferably within the range of about 130°C to about 145°C, favor the production of 1-pentene and hinder the production of 2-pentene. One-pentene is more desirable in that it can be polymerized alone, or more importantly, be used as a comonomer to polymerize with other mono-1-olefins, such as, for example, ethylene and/or propylene.

The dimerization reaction can be carried out by contacting the olefins with catalyst in the liquid phase or the gas phase, depending on the structure and molecular weight of the olefin, as well as reaction temperature and pressure employed. Pressure during the dimerization reaction can vary between wide limits. In general, higher pressures favor the progress of the reaction. Thus, pressures of atmospheric up to about 10,000 psig and higher are suitable. Preferably, pressures of about 100 to about 5,000 psig are employed, with pressure of about 1,000 to about 4,000 psig most preferred in order to achieve a good balance between reaction rate and minimize equipment and operating costs necessitated by very high reaction pressures.

If the reaction is carried out in the liquid phase, solvents or diluents for the reactants can be used. Saturated aliphatic hydrocarbons, e.g., pentane, hexane, cyclohexane, dodecane; aromatic compounds, preferably those without an alpha-hydrogen (which would be capable of undergoing alkylation under the reaction conditions) such as benzene and chlorobenzene are suitable. If the reaction is carried out in the gaseous phase, diluents such as aliphatic hydrocarbons, for example methane, ethane and/or substantially inert gases, e.g., nitrogen, argon, can be present.

The contact time required for the dimerization reaction depends upon several factors such as for example the activity of the catalyst, temperature, pressure, structure of the reactants employed, level of conversion desired, and the like. The length of time during which the dimerizable olefinic compounds are contacted with catalyst can vary conveniently between about 0.1 seconds and about 24 hours although shorter and longer contact times can be employed. Preferably, times of about one minute to about 5 hours are employed. Where reaction is carried out in continuous fashion, it is convenient to express the reactant/catalyst contact time in terms of weight hourly space velocity (WHSV), i.e., the ratio of the weight of reactant which comes in contact with a given weight of catalyst per unit time. Thus, a WHSV of about 0.1 to about 10 will be employed. A WHSV of about 0.5 to about 5 is preferred, with about 1 to about 4 WHSV most preferred for optimum catalyst productivity.

## Products

The olefinic products of the invention have established utility in a wide variety of applications such as for example as monomers for use in the preparation of homopolymers, copolymers, terpolymers, e.g., as the third component of ethylene-propylene terpolymers useful as synthetic elastomers, and the like. The preferred olefinic products of the invention are mono-1-olefins, such as, for example, 1-pentene and/or 4-methyl-1-pentene. These mono-1-olefins can be polymerized either alone to form polymers, such as, for example, poly(4-methyl-1-pentene) or used as comonomers in other olefin polymerization processes. For example, 1-pentene and/or 4-methyl-1-pentene can be used as excellent comonomers with ethylene and/or propylene polymerization processes.

The preferred olefinic products of this invention, 1-pentene and 4-methyl-1-pentene, can be produced in a weight ratio of 1-pentene to 4-methyl-1-pentene within the range of about 20:1 to about 0.2:1, depending on the ratio of propylene to ethylene and the reaction temperature. Preferably, the weight ratio of 1-pentene to 4-methyl-1-pentene is within the range of about 15:1 to about 0.5:1.

A further understanding of the present invention and its advantages will be provided by reference to the following examples.

## Examples

In each of the following examples, typically, the dimerization of propylene was carried out in a steam heated 316 stainless steel tubular reactor ($\frac{1}{2}$ " X 20"). The catalyst system (27 grams; density about 0.84 g/mL), bounded above and below by small volumes of glass beads, was combined with 25 grams of an inert substance, i.e., no dimerization catalytic activity, to dilute the catalyst system and thus reduce and control the reaction rate. The contents of the tubular reactor were heated to the reaction temperature of about 160°C at about 1500 psig and propylene was pumped into the reactor at a rate of about 120 mL/hr. After about 1.5 hours of reaction time and each one hour thereafter for the following 6 hours, a sample was collected and analyzed by gas liquid chromatography (glc). The summarized results represent the analysis of the last dimerization sample collected.

Extruded catalyst support was prepared from commercially available, anhydrous potassium carbonate (JT Baker, ACS reagent grade), 1-propanol (n-propyl alcohol) (ACS reagent grade), and deionized water. 1-propanol was premixed with potassium carbonate, particle size of equal to or less than about 0.42 mm (40 mesh), to wet the potassium carbonate; then water was added. The catalyst support did not contain any graphite. The thick paste was thoroughly mixed and extruded through a die plate with 1/8" openings in a single screw, $2\frac{1}{4}$ " Bonnot extruder. The extrudate was collected and allowed to break into pieces; each piece was from about 1/4" to about 7/8" long.

The extrudate was dried at about 150°C in a vacuum oven for at least 2 hours. The dried extrudate was then calcined for about 3 hours in an oxygen-containing atmosphere at a temperature of at least about 250°C and transferred to a dry, oxyen-free atmosphere. The resultant support was maintained at a temperature of about 80° to about 85°C, in an oxygen-free atmosphere, at which time about 8 weight percent of elemental potassium and about 5 weight percent of finely divided 316 stainless steel (about 325 mesh). At all times after calcination, the catalyst support and catalyst system were kept under a dry, inert atmosphere.

The results of the corresponding dimerization reactions are summarized in Table 1.

Table I

| Run | 1 | 2 | 3 | 4 | 5 | 6 |
|---|---|---|---|---|---|---|
| Propylene/Ethylene Weight (g) Ratio | 3:1 | 3:1 | 3:1 | 6:1 | 12:1 | 12:1 |
| Propylene/Ethylene Mole Ratio | 2:1 | 2:1 | 2:1 | 4:1 | 8:1 | 8:1 |
| Reactor Temp., °C | 160 | 150 | 140 | 140 | 160 | 150 |
| Product Analysis, Weight (g) %* | | | | | | |
| 1-Pentene | 34.4 | 35.3 | 28.6 | 18.4 | 11.8 | 12.4 |
| 2-Pentene | 12.0 | 8.6 | 2.7 | 1.6 | 3.6 | 2.5 |
| 4MP1 | 8.6 | 6.8 | 2.3 | 2.2 | 16.7 | 11.9 |
| other hexenes** | N/A | 1.2 | 0.4 | 0.3 | N/A | 1.6 |

\* Unreacted propylene and ethylene are also present in the product stream.
\*\* Includes, but not limited to, 4-methyl-2-pentene, 1-hexene, and/or 2-methyl-2-pentene.
N/A = not analyzed.

The data in Table I show that propylene and ethylene can be dimerized in the presence of an elemental alkali metal supported on an alkali metal carbonate catalyst. Furthermore, the data confirms that the reaction is not stoichiometric. Comparison of Run 1 to Run 5, Run 2 to Run 6, and Run 3 to Run 4 shows that varying the reactant ratio significantly effects the types of products produced. Examination of Runs 1, 2, and 3, as well as Runs 5 and 6, indicate that lower reactor temperatures favor the production of 1-pentene over 2-pentene.

The examples have been provided merely to illustrate the practice of the invention and should not be read so as to limit the scope of the invention or the appended claims in any way. Reasonable variations and modifications, not departing from the essence and spirit of the invention, are contemplated to be within the scope of patent protection desired and sought.

**Claims**

1. A process for preparing olefin dimerization products comprising:
reacting propylene and ethylene under dimerization conditions in the presence of a catalyst comprising an elemental alkali metal supported on an alkali metal corbonate to produce mono-1-olefin reaction products, characterized in that the type and quantity of said reaction product is controlled by varying the ratio of propylene to ethylene and the dimerization reaction conditions.

2. The process of claim 1 wherein said reaction conditions comprise a temperature within the range of 50 to 250 °C.

3. The process of claim 1 or 2 wherein said reaction conditions comprise a pressure within the range of atmospheric pressure to 69 MPa.

4. The process of any of the preceding claims wherein said reaction is carried out at a weight hourly space velocity in the range of 0.1 to 10.

5. The process of any of the preceding claims wherein said elemental alkali metal is selected from lithium, sodium, potassium, and mixtures thereof.

6. The process of claim 5 wherein said elemental alkali metal is potassium.

7. The process of any of the preceding claims wherein said alkali metal carbonate is selected from sodium carbonate, potassium carbonate, and mixtures thereof.

8. The process of claim 7 wherein said alkali metal carbonate is potassium carbonate.

9. The process of any of the preceding claims wherein said mono-1-olefin reaction products are selected from 1-pentene, 4-methyl-1-pentene, and mixtures thereof.

10. The process of any of the preceding claims wherein said propylene to ethylene mole ratio is within the range of 0.3:1 to 10:1.

11. The process of any of claims 1-9 wherein said propylene to ethylene weight ratio is within the range of 0.5:1 to 15:1.

## DOCUMENTS CONSIDERED TO BE RELEVANT

EP 90107085.4

| Category | Citation of document with indication, where appropriate, of relevant passages | Relevant to claim | CLASSIFICATION OF THE APPLICATION (Int Cl⁵) |
|---|---|---|---|
| X | GB - A - 1 249 697 (BP CHEMICALS) * Claims 1-19,21-23; examples 1-4; page 1, lines 86-90 * | 1-3, 4-6, 9-11 | C 07 C 2/24 C 07 C 11/10 C 07 C 11/113 |
| Y | US - A - 4 544 790 (DRAKE) * Claims; example XII; column 4, lines 46-57 * | 1-11 | |
| Y | US - A - 4 687 877 (BARTLEY et al.) * Example 1 * | 1-11 | |
| A | DE - A - 1 618 176 (BRITISCH PETROLEUM) * Claims; examples * | 1-3,4- 11 | |

TECHNICAL FIELDS SEARCHED (Int Cl⁵)

C 07 C 2/00
C 07 C 11/00

The present search report has been drawn up for all claims

| Place of search | Date of completion of the search | Examiner |
|---|---|---|
| VIENNA | 12-07-1990 | KÖRBER |